**Europäisches Patentamt**

**European Patent Office**

**Office européen des brevets**

(19)

(11) Veröffentlichungsnummer: **0 169 976**
**B1**

(12)

# EUROPÄISCHE PATENTSCHRIFT

(45) Veröffentlichungstag der Patentschrift:
**05.10.88**

(51) Int. Cl.⁴: **A 61 F 2/36**

(21) Anmeldenummer: **85104237.4**

(22) Anmeldetag: **06.04.85**

(54) **Sich vom distalen Ende konisch erweiternder Schaft für eine Hüftgelenksprothese.**

(30) Priorität: **03.07.84 CH 3184/84**

(43) Veröffentlichungstag der Anmeldung:
**05.02.86 Patentblatt 86/6**

(45) Bekanntmachung des Hinweises auf die Patenterteilung:
**05.10.88 Patentblatt 88/40**

(84) Benannte Vertragsstaaten:
**AT DE FR GB IT**

(56) Entgegenhaltungen:
**WO - A - 83/02555**
**BE - A - 493 526**
**CH - A - 560 042**
**DE - A - 2 839 093**
**US - A - 3 067 740**

(73) Patentinhaber: **GEBRÜDER SULZER AKTIENGESELLSCHAFT, Zürcherstrasse 9, CH-8401 Winterthur (CH)**

(72) Erfinder: **Griss, Peter, Prof. Dr., Klinikum Mannheim, D-6800 Mannheim (DE)**

(74) Vertreter: **Dipl.-Ing. H. Marsch Dipl.-Ing. K. Sparing Dipl.-Phys.Dr. W.H. Röhl Patentanwälte, Rethelstrasse 123, D-4000 Düsseldorf (DE)**

## Beschreibung

Die Erfindung betrifft einen sich vom distalen Ende konisch erweiternden Schaft für eine Hüftgelenksprothese, der mindestens im proximalen Bereich seiner im eingebauten Zustand im wesentlichen nach anterior/posterior weisenden Oberflächenteile mit Längsrippen versehen ist, die mindestens im wesentlichen in Richtung der Schaftlängsachse verlaufen, wobei die Höhe der Rippen höchstens 1/4 der maximalen Schaftdicke, gemessen in Richtung anterior/posterior zwischen den Rippenscheitelpunkten, beträgt, und der Abstand zweier benachbarter Rippenscheitelpunkte mindestens gleich der Rippenhöhe ist.

Prothesenschäfte der genannten Art sind z.B. aus der DE-A-23 56 464 bekannt; bei der dortigen Konstruktion erfolgt die Fixierung des Schaftes, über die die Weiterleitung der wesentlichen Belastungskräfte erfolgt, vorwiegend im distalen Bereich, wobei die Rippenstruktur im proximalen Bereich in erster Linie als Rotationssicherung dient.

Weiterhin ist aus der DE-A-32 16 539 eine ähnliche Schaftkonstruktion bekannt, bei der in ihrer Höhe die vorstehenden Abmessungen übersteigende Rippen auf dem Umfang verteilt sind. Dieser Schaft wird primär ebenfalls im distalen Bereich fixiert; zwischen die und durch die mit Durchbrüchen versehenen Rippen hindurch soll im Laufe der Zeit spongiöses Gewebe einwachsen, das zuvor bei der Implantation ausgeräumt worden ist. Das zwischen die Rippen einwachsende Gewebe dient dabei als Rotationssicherung und zur Verbesserung der Sekundärfixierung.

In neuerer Zeit ist man dazu übergegangen, die Verankerungsschäfte von Femurkopfprothesen im intertrochantären Bereich zu fixieren, um das auf die Fixierung durch die einseitige, auf den Gelenkkopf wirkende Belastung ausgeübte Biegemoment möglichst gering zu halten.

Eine wirkungsvolle Massnahme zur Fixierung im proximalen bzw. intertrochantären Bereich besteht darin, den Schaft von distal nach proximal konisch erweitert zu gestalten und in spongiöses Gewebe, das im operativ vorbereiteten Knochen verblieben ist, einzutreiben, um ihn in einem verdichteten Bett aus diesem Gewebe zu lagern. Dabei ist einerseits eine notwendige Minimalverdichtung der Spongiosa gegeben, während auf der anderen Seite die bei der Verdichtung auftretenden radialen Kräfte nicht die Gefahr einer Sprengung des kortikalen Knochens in Umfangsrichtung auslösen dürfen. Aus diesem Grund ist die Einhaltung der eingangs erwähnten, an sich bekannten Abmessungen für die Rippenstruktur erforderlich. Ein zusätzlicher Vorteil der vorstehend beschriebenen Art der Verankerung sind eine erhöhte Primärstabilität und ein rasches Ein- und Zusammenwachsen des verdichteten Gewebes, da verdichtetes Gewebe – wie man weiss – sehr rasch heilt.

Aufgabe der vorliegenden Erfindung ist es, die Sekundär-Fixierung durch ein- und anwachsendes Gewebe zu beschleunigen und zu verbessern. Diese Aufgabe wird erfindungsgemäss dadurch gelöst, dass in die Längsrippenstruktur im proximalen Bereich Quernuten eingearbeitet sind, die mit der vertikalen Längsachse des Schaftes von der Horizontalen abweichende unterschiedliche Winkel bilden.

Die Quernuten bilden «Kanäle» zwischen den einzelnen Längsrippen, so dass das verdichtete Gewebe – oder gegebenenfalls auch ein Knochenzementbett – nicht nur in den Längsrippen verteilt ist, sondern sich auch quer dazu ausbreiten kann. Auf diese Weise bilden sich zwischen den einzelnen Längsrippen Gewebe- oder Zementbrücken, durch die ein fixierendes «Netz» entsteht. Die Winkelstellung der Quernuten gegenüber der Längsachse entspricht dabei wenigstens annähernd der Richtung der bei der Heilung wachsenden Spongiosa-«Bälkchen», die sich bekanntlich jeweils in Richtung der örtlichen Belastungen des Gewebes ausbilden. Daher ist es zweckmässig, wenn sich die Winkel von distal gelegenen Quernuten zu den proximal gelegenen verringern.

Um beim Eintreiben des Schaftes zusätzlich spongiöses Gewebe abzuschaben, das sich in den Nuten verdichtet, ist es weiterhin vorteilhaft, wenn die Unterkanten der durch die Quernuten an den Längsrippen entstehenden Zähne als scharfkantige Schneiden ausgebildet sind.

Im folgenden wird die Erfindung anhand eines Ausführungsbeispiels im Zusammenhang mit der Zeichnung näher erläutert.

Fig. 1 zeigt den proximalen Bereich eines Schaftes für eine Hüftgelenksprothese in einer Aufsicht auf die nach anterior oder posterior weisende Oberfläche;

Fig. 2 ist ein Schnitt II–II von Fig. 1 in einem vergrösserten Mass-Stab, während

Fig. 3 der Schnitt III–III von Fig. 1 ist.

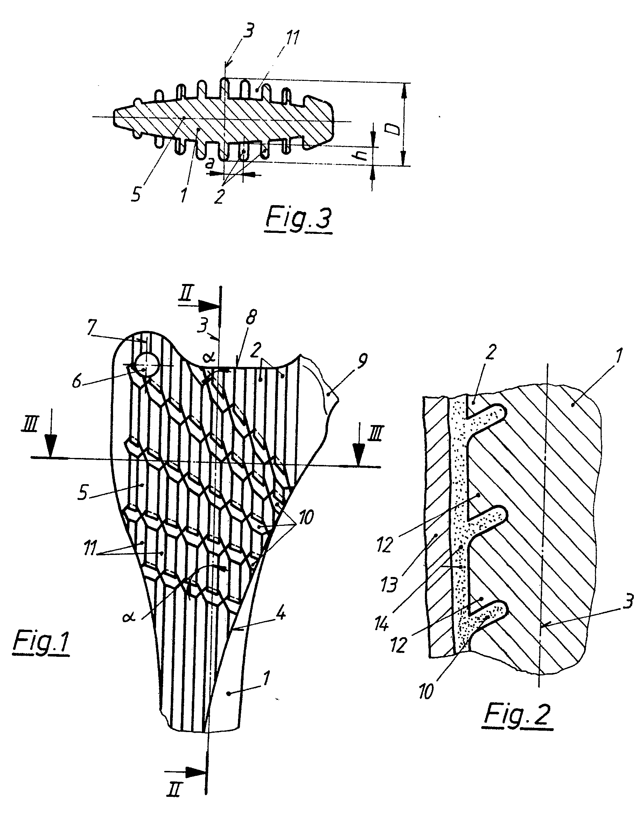

Der Schaft 1 (Fig. 1) ist in seinem proximalen Bereich mit einer aus Längsrippen 2 bestehenden Oberflächenstruktur auf der mit der Zeichenebene zusammenfallenden Oberfläche versehen. Die Längsrippen 2 verlaufen parallel zur Längsachse 3 des Schaftes 1 und enden auf der Seite medial von dieser Achse 3 in einem nicht weiter dargestellten Absatz 4.

In den Schaft 1 ist ein Trochanterflügel 5 integriert, der nach lateral aus dem Schaft 1 hervorspringt und über eine mit einer Ausziehöse 6 versehene Erhöhung 7 in eine horizontale Schulter 8 übergeht. An diese schliesst sich – in Fig. 1 nach rechts – ein nur im Ansatz gezeigter Prothesenhals 9 an, der den nicht dargestellten Gelenkkopf trägt.

Die Höhe h der Rippen 2 beträgt etwa 1/5 der Schaftdicke D gemessen vom Rippenscheitelpunkt zu Rippenscheitelpunkt einander gegenüberliegender nach anterior bzw. posterior gerichteter Rippen 2. Ferner ist der Abstand a zweier Rippenscheitelpunkte mindestens gleich ihrer Höhe h.

Erfindungsgemäss sind die Längsrippen 2 von Quernuten 10 durchzogen, die mit der Längs-

achse 3 spitze Winkel α bilden. Die Beträge der Winkel α nehmen beim Fortschreiten von einer distalen zu einer proximalen Quernute 10 ab. Auf diese Weise bilden die Quernuten 10 zusammen mit den Zwischenräumen 11 zwischen den Rippen 2 ein netzartiges Muster. In dieses einwachsendes Gewebe erhält dadurch einen Aufbau, der dem «Gittersystem» natürlicher Spongiosa-«Bälkchen» ähnelt.

Wie Fig. 2 zeigt, entstehen durch die Quernuten 10, die zu ihrem Grund hin leicht ansteigen, so dass sie mit der Längsachse 3 unter einem von 90° verschiedenen Winkel verlaufen, an den Rippen 2 scharfkantige Schneiden 12; diese schälen beim Eintreiben des Schaftes 1 in den Knochen 13 spongiöses Gewebe 14 ab, wobei der «Schälspan» in die Quernut 10 hinein verdrängt wird. Dabei erfolgt eine intensive Verdichtung der entstehenden Knochenspäne.

## Patentansprüche

1. Sich vom distalen Ende konisch erweiternder Schaft für eine Hüftgelenksprothese, der mindestens im proximalen Bereich seiner im eingebauten Zustand im wesentlichen nach anterior/posterior weisenden Oberflächenteile mit Längsrippen versehen ist, die im wesentlichen in Richtung der Schaftlängsachse verlaufen, wobei die Höhe der Rippen höchstens 1/4 der maximalen Schaftdicke, gemessen in Richtung anterior/posterior zwischen den Rippenscheitelpunkten, beträgt, und der Abstand zweier benachbarter Rippenscheitelpunkte mindestens gleich der Rippenhöhe ist, dadurch gekennzeichnet, dass in die Längsrippenstruktur (2) im proximalen Bereich Quernuten (10) eingearbeitet sind, die mit der vertikalen Längsachse (3) des Schaftes (1) von der Horizontalen abweichende unterschiedliche Winkel (α) bilden.

2. Schaft nach Anspruch 1, dadurch gekennzeichnet, dass sich die Winkel (α) von distal gelegenen Quernuten (10) zu den proximal gelegenen verringern.

3. Schaft nach Anspruch 1 oder 2, dadurch gekennzeichnet, dass die Unterkanten der durch die Quernuten (10) an den Längsrippen (2) entstehenden Zähne als scharfkantige Schneiden (12) ausgebildet sind.

## Revendications

1. Queue s'élargissant coniquement à partir de l'extrémité distale, pour prothèse coxo-fémorale, qui, au moins dans la zone proximale de sa partie de surface d'orientation sensiblement antérieure/postérieure, dans l'état encastré, est munie de nervures longitudinales qui s'étendent sensiblement dans la direction de l'axe longitudinal de la queue, la hauteur des nervures atteignant au maximum un quart de l'épaisseur maximale de la queue, mesurée dans la direction antérieure/postérieure entre les sommets des nervures, et l'écartement entre deux sommets de nervures voisines étant au moins égal à la hauteur des nervures, caractérisée en ce que la zone proximale de la structure à nervures longitudinales (2) présente des rainures transversales (10) qui forment, avec l'axe longitudinal vertical (3) de la queue (1), des angles (α) différents s'écartant de l'horizontale.

2. Queue selon la revendication 1, caractérisée en ce que les angles (α) diminuent des rainures transversales (10) du côté distal vers celles du côté proximal.

3. Queue selon la revendication 1 ou 2, caractérisée en ce que les bords inférieurs des dents résultant de la présence des rainures transversales (10) dans les nervures longitudinales (2) sont réalisés sous forme de lames (12) à arête vive.

## Claims

1. A shank, widening conically from the distal end, for a hip joint prosthesis and formed with longitudinal ribs at least in the proximal region of its surface parts which, when fitted, extend substantially in the anterior/posterior direction, the ribs extending substantially in the direction of the longitudinal axis of the shank and the height of the ribs being not more than a quarter of the maximum thickness of the shank measured in the anterior/posterior direction between the rib apices, and the distance between two adjacent rib apices being at least equal to the rib height, characterised in that the longitudinal rib structure is formed in the proximal region with transverse grooves (10) at various non-horizontal angles (α) to the vertical longitudinal axis (3) of the shank (1).

2. A shank according to claim 1, characterised in that the angles (α) decrease from the distal to the proximal transverse grooves (10).

3. A shank according to claim 1 or 2, characterised in that the lower edges of the teeth formed by the transverse grooves (10) on the longitudinal ribs (2) constitute sharp edges (12).

1/1

Fig. 3

Fig. 1

Fig. 2